Europäisches Patentamt

European Patent Office

Office européen des brevets

(11) Publication number: **0 122 630**
**B1**

(12) **EUROPEAN PATENT SPECIFICATION**

(45) Date of publication of patent specification: **07.02.90**

(51) Int. Cl.⁵: **A 61 M 16/00**

(21) Application number: **84104265.8**

(22) Date of filing: **14.04.84**

(54) Apparatus for providing or improving respiratory aids.

(30) Priority: **15.04.83 IT 2062383**

(43) Date of publication of application:
**24.10.84 Bulletin 84/43**

(45) Publication of the grant of the patent:
**07.02.90 Bulletin 90/06**

(84) Designated Contracting States:
**AT BE CH DE FR GB IT LI NL SE**

(56) References cited:
**GB-A-2 063 686**
**GB-A-2 105 594**
**US-A-4 265 237**

(73) Proprietor: **Bertocchi, Giacomo**
**Via De' Berenzani, 8a/b/c**
**I-26100 Cremona (IT)**

(72) Inventor: **Bertocchi, Giacomo**
**Via Cavour, 14**
**I-26100 Cremona (IT)**
Inventor: **Marini, Antonio**
**Via Commenda, 12**
**I-20100 Milan (IT)**
Inventor: **Pagani, Giovanni**
**Via Marconi, 2/c**
**I-22070 Appiano Gentile (Como) (IT)**

(74) Representative: **Dr. Ing. A. Racheli & C.**
**Viale San Michele del Carso, 4**
**I-20144 Milano (IT)**

Courier Press, Leamington Spa, England.

**Description**

The invention relates to a process and relative apparatus for improving the quality of a patient's respiratory aids. The present apparatuses, normally referred to as pulmonary ventilators, are used both in human medicine and veterinary science. The respiratory pathologies wherein the pulmonary ventilator is used for treatment are of many different kinds. At present, these types of pulmonary ventilators are used:

CPAP: Continuous Positive Airway Pressure. By this technique, a patient spontaneously breathing has applied a constant positive pressure both in breathing in and out.

IMV: Intermittent Mandatory Ventilation. The ventilator compels a spontaneously breathing patient to periodical breaths of predetermined length and frequency.

IPPV (IPPB): Intermittent Positive Pressure Ventilation (Intermittent Positive Pressure Breathing). The ventilator compels a patient to breathe with a preselected frequency and flow, when incapable of doing so by himself.

These pulmonary ventilators at present commercially available either bind a patient to the mechanical type of breathing selected by the medical staff on the basis of known techniques and/or put the lungs of a patient under a too high PEEP (Positive End Expiratory Pressure). In such cases, an alveolar pressure higher than the atmospheric pressure is maintained by means of water valves or other valves applied to the expiration duct.

Recently, some researchers have realized the possibility of carrying oxygen into the pulmonary alveoli of patients by taking advantage of a technique imposing no respiratory rhythm.

These techniques are identified by the term High Frequency Ventilation (HFV), and among them the so-called "Oscillatory" technique HFOV (High Frequency Oscillation Ventilation), in which pressure peaks are created by closing and opening, for example, a valve placed on the main flow, or acting on the same flow by means of a piston. This kind of ventilation submits the patient to a MAP (Mean Airway Pressure) that he cannot always tolerate.

Another type of HFV is the "jet" ventilation or the HFJV (High Frequency Jet Ventilation) according to which a low flow intermittent jet is sent into a feeding duct. Although the short term experiments were positive, it has been hitherto found that these new techniques give rise to several problems, if used continuously.

US—A—4.265.237 describes an apparatus for improving or providing respiratory assistance, substantially formed by a jet ventilator comprising a pressure wave detector for continuously detecting the actual pressure inside the patient's respiratory canals and a frequency control device which modifies the jet cycle frequency and the ratio between the on-and off periods.

Also this apparatus gives rise to problems when used continuously.

Therefore, it is an object of the invention to provide a ventilation imposing on a patient no substantial respiratory rhythm and capable of being used for extended periods of time.

The above object has been achieved as specified in claim 1.

Other advantages are described in claim 2.

The present invention will now be further explained by an exemplary embodiment to be hereinafter described with reference to the accompanying schematic drawings, in which:

Fig. 1 is a block diagram of the proposed apparatus;

Fig. 2 is a view showing several patterns for the pressure wave as provided for ratios of feeding to closing time in the range of between 1:4 and 4:1;

Fig. 3 is a view showing a pressure pattern in a patient's aorta for a curve obtained with a ratio of 1:4;

Fig. 4 is a view showing the pressure wave pattern in the trachea under conditions similar to the previous ones;

Fig. 5 is a front view of the proposed connector for the apparatus shown in Fig. 1; and

Fig. 6 is a top view of the latter.

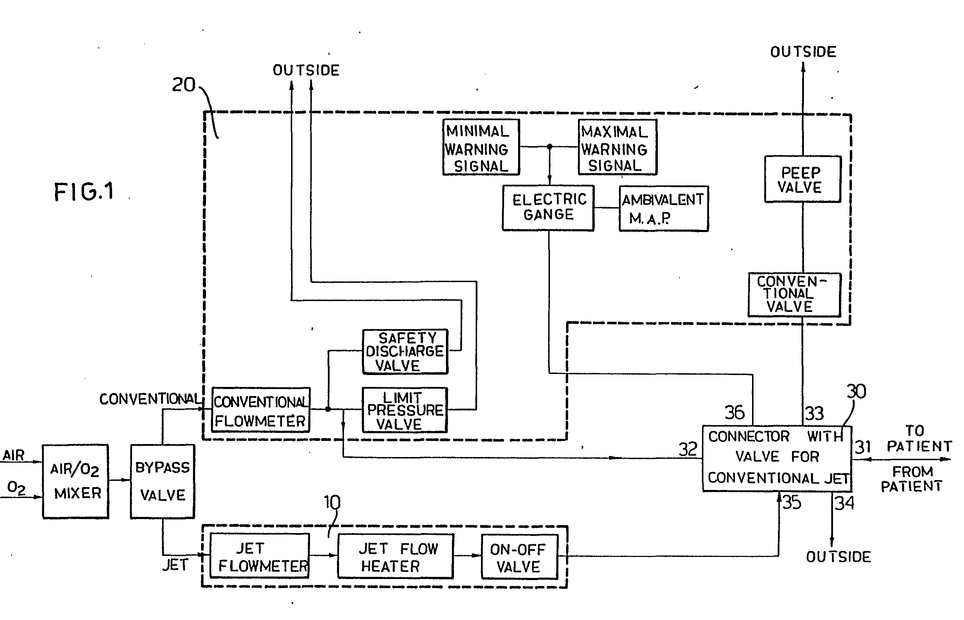

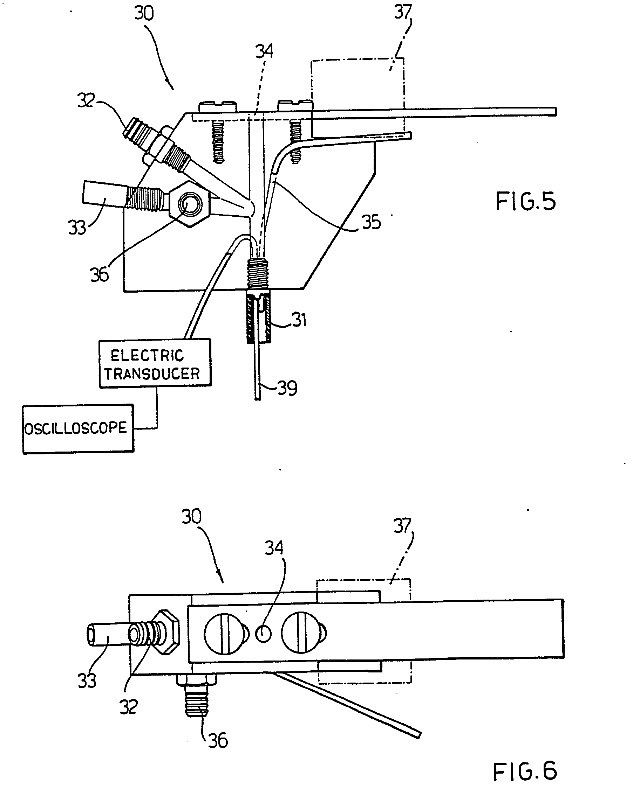

Referring first to Fig. 1, it will be seen that oxygen and air are sent to a mixer, then they arrive at a by-pass valve sending this mixture either to the jet ventilation 10, or to a conventional ventilator 20. As well known, the latter comprises a flowmeter sending air to the connector 30, the latter being shown in Figs. 5 and 6. This pipe has connected thereto a safety discharge valve, a limit pressure valve and an electric pressure gauge capable of giving a minimal and maximal warning signal. A branching off 36 leads to the sensing of the average alveolar pressure (MAP Mean airway pressure). The endotracheal tube 31 is connected at its outlet 34 with a known PEEP valve. Also a small duct 39 reaches the endotracheal canal 31, the internal end of said duct 39 being placed some centimeters beyond the jet duct end. The other end of duct 39 is connected to a transdcer and then to an oscillator detecting the pressure wave form.

The jet apparatus 10 sequentially comprises a flowmeter, a flow heater, and a high frequency alternatively operating on-off valve sending the alternate flow into the endotracheal canal in the patient's direction through a nozzle having a diameter in the range of between 0.5 and 1.5 mm. Particularly, the connector 30 has two positions: a first position connects the conventional ventilator to the endotracheal canal through the metal tube 31, or joins the inlets 32 and 33 with said tube 31, while MAP pressure is being monitored through the connection 36. The second position of the connector, controlled by the electromagnet 37, connects said metal tube 31 with ambient through the hole 34 while the inside pressure and the wave form are monitored through the tube 34 by means of the oscilloscope. Of course, the electric circuit is such that at each of the two positions for the connector 30, the inlet 32 and outlet 33 are respectively cut off.

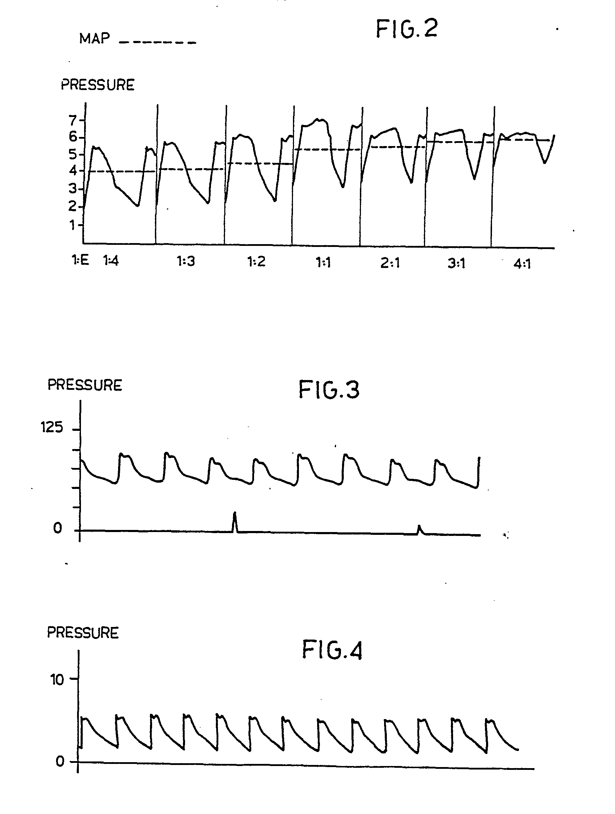

When in use the operator will control the apparatus' variables so that a pressure wave will have the form shown in Figure 4. Usually this form is reach with on-off periods ratio comprised between 1:2 and 1:4. However the pressure wave form is defined by the patient too, so no predetermined position of the apparatus can be used.

The pressure wave provided by the jet apparatus, particularly by the on-off valve has the pattern shown in Figure 2 for several types of alternances, wherein the feeding time is related to the closing time, that is the I/E ratio is in the range of between 1:4 and 4:1.

As said above the apparatus will have to be controlled so that the pressure wave inside the patient's respiratoy canals will result as similar as possible to the one represented in Figure 4.

This is achieved in a different way according to each patient.

Corresponding waves in aorta are shown in Figure 3.

## Claims

1. An apparatus for improving or providing respiratory assistance, comprising a jet ventilator (10) having a pressure wave detector for continuously detecting the actual pressure inside the patient's respiratory canals and a frequency control device which modifies the jet cycle frequency and the ratio between the on and off periods, characterized in that the apparatus further comprises a conventional IPPB ventilator (20) connected to the jet ventilator (10) through a by-pass valve placed downstream of an air-oxygen mixer and a connector (30) having two positions: the first one receiving the alternating jet and sending it to the patient, while connecting at the same time the endotracheal canal with the ambient or a PEEP valve; the second one connecting the endotracheal canal with the inlet and the outlet of the conventional IPPB ventilator.

2. An apparatus according to claim 1, characterized in that the connecting tubes between the jet valve and the patient's lungs are made of a rigid material having a very low "compliance".

## Patentansprüche

1. Vorrichtung zur Verbesserung und Verwirklung der Atmungsbehandlung mit einem Strahlgebläse (10), das einen Druckwellendetektor zur kontinuierlichen Aufnahme des effektiven Drucks im Innern der Atmungskanäle des pazienten und eine Frequenzsteuervorrichtung, die die Frequenz des Strahlzyklus und das Verhältnis zwischen den Auslaß-und Schließzeiten verändert, aufweist, dadurch gekennzeichnet, daß die Vorrichtung außerdem ein herkömmliches Gebläse IPPB (20) aufweist, das mit dem Strahlgebläse (10) durch ein hinter dem Luft-Sauerstoff-Mischer angebrachtes Umlaufventil verbunden ist, und ein Verbindungselement (30) mit zwei Stellungen, wobei die erste Stellung den alternierenden Strahl empfängt und an den Pazienten weiterleitet und gleichzeitig den inneren Luftröhrenkanal mit der Umgebung oder mit einem PEEP-Ventil verbindet und die zweite Stellung den Luftröhrenkanal mit dem Eingang und Ausgang des herkömmlichen IPPB-Gebläses verbindet.

2. Vorrichtung nach Anspruch 1, dadurch gekennzeichnet, daß die Verbindungsrohre zwischen dem Strahlventil und den Lungen des Pazienten aus steifem Material mit geringster "compliance" verwirklicht sind.

## Revendications

1. Dispositif d'amélioration d'aides respiratoires comprenant un ventilateur du type à jet (10) présentant un détecteur d'ondes de pression permettant de relever constamment la pression effective à l'intérieur de canaux respiratoires du patient et un dispositif de commande de fréquence modifiant la fréquence du cycle du jet et le rapport entre les temps d'ouverture et de fermeture, caractérisé en ce que le dispositif comprend en outre un ventilateur traditionnel du type IPPB (20) relié au ventilateur à jet à l'aide d'une soupape d'introduction directe (by-pass) placée en aval d'un mélangeur d'air-oxygène et un connecteur (30) à deux positions: la première pour recevoir le jet alterné et l'envoyer au patient, tandis qu'est simultanément mis en liaison le canal endotrachéal avec l'air ambiant ou avec une soupape PEEP; la seconde qui met en liaison le canal endotrachéal avec l'entrée et la sortie du ventilateur traditionnel IPPB.

2. Dispositif selon la revendication 1, caractérisé en ce que les tubes de liaison entre la soupape jet et les poumons du patient sont réalisés en matériau rigide ayant une "compliance" fort réduite.

FIG.1

FIG. 2

MAP _ _ _ _ _ _

PRESSURE

1:E  1:4    1:3    1:2    1:1    2:1    3:1    4:1

PRESSURE

FIG. 3

125

0

PRESSURE

FIG. 4

10

0

FIG.5

ELECTRIC
TRANSDUCER

OSCILLOSCOPE

FIG.6